(19) Europäisches Patentamt

European Patent Office

Office européen des brevets

(11) Veröffentlichungsnummer: **0 451 810 B1**

(12) **EUROPÄISCHE PATENTSCHRIFT**

(45) Veröffentlichungstag der Patentschrift: **16.08.95**

(21) Anmeldenummer: **91105678.6**

(22) Anmeldetag: **10.04.91**

Die Akte enthält technische Angaben, die nach
dem Eingang der Anmeldung eingereicht wurden
und die nicht in dieser Patentschrift enthalten sind.

(51) Int. Cl.⁶: **G01N 33/531**, G01N 33/536,
G01N 33/74, G01N 33/53,
A61K 47/48, //A61K31/52,
A61K31/415

(54) **Hapten-Biotin-Konjugate und ihre Verwendung.**

(30) Priorität: **10.04.90 DE 4011601**

(43) Veröffentlichungstag der Anmeldung:
**16.10.91 Patentblatt 91/42**

(45) Bekanntmachung des Hinweises auf die
Patenterteilung:
**16.08.95 Patentblatt 95/33**

(84) Benannte Vertragsstaaten:
**AT BE CH DE DK ES FR GB GR IT LI LU NL SE**

(56) Entgegenhaltungen:
**EP-A- 0 183 901**
**EP-A- 0 310 361**
**EP-A- 0 315 317**
**EP-A- 0 349 988**

(73) Patentinhaber: **BOEHRINGER MANNHEIM
GMBH**

**D-68298 Mannheim (DE)**

(72) Erfinder: **Huber, Erasmus, Dr.
St. Willibald 10**
**W-8911 Unterfinning (DE)**
Erfinder: **Zdunek, Dietmar, Dr.
Blütenstrasse 21
W-8000 München 40 (DE)**
Erfinder: **Klein, Christian, Dr.
Blütenstrasse 16
W-8120 Weilheim (DE)**
Erfinder: **Schenk, Roland, Dr.
Bärenmühlweg 64
W-8120 Weilheim (DE)**

(74) Vertreter: **Huber, Bernhard, Dipl.-Chem. et al
Patentanwälte
H. Weickmann, Dr. K. Fincke
F.A. Weickmann, B. Huber
Dr. H. Liska, Dr. J. Prechtel, Dr. B.
Böhm
Postfach 86 08 20
D-81635 München (DE)**

EP 0 451 810 B1

EP 0 451 810 B1

## Beschreibung

Die Erfindung betrifft Hapten-Biotin-Konjugate und ihre Verwendung.

In Körperflüssigkeiten und Geweben kommen viele Substanzen vor, die mit einem spezifischen Bindungspartner bindefähig sind, selbst aber keine immunologische Reaktion auslösen können und daher als Haptene zu bezeichnen sind, die als Parameter für bestimmte Erkrankungen oder den Gesundheitszustand des menschlichen Körpers dienen. Hierzu zählen unter anderem Hormone, Tumormarker und virale Proteine. Den Haptenen zuzurechnen sind weiterhin die meisten Arzneistoffe, deren Bestimmung für die Überwachung einer medikamentösen Behandlung oft erforderlich ist. Da alle diese Haptene nur in sehr geringen Mengen vorkommen, verwendet man zu ihrem Nachweis Verfahren nach dem Prinzip des Immunoassays. Dazu gibt es viele Varianten. Die verschiedenen immunologischen Bestimmungsverfahren lassen sich in homogene und heterogene Verfahren einteilen. Bei den heterogenen Verfahren ist immer eine Festphasenreaktion beteiligt, um den gebundenen Anteil der markierten Komponenten von dem ungebundenen abzutrennen. Bei dieser Verfahrensart läßt sich die Markierung gut bestimmen, nachteilig ist jedoch, daß die heterogene Reaktion lange dauert.

Bei der homogenen Verfahrensvariante erfolgt keine Trennung von gebundener Markierung und ungebundener Markierung, so daß eine Differenzierung von gebundener und ungebundener Markierung durch andere Methoden erfolgen muß. Hierzu gibt es verschiedene Möglichkeiten. So können beispielsweise konjugierte Enzyme als Markierung verwendet werden, die nur dann ihre Enzymaktivität erhalten, wenn sie an das zu bestimmende Hapten oder Antigen gebunden werden oder durch die zu bestimmende Substanz aktiviert werden. Eine weitere Möglichkeit besteht darin, als Markierung eine fluoreszierende Substanz zu verwenden, deren Fluoreszenz durch Bindung an die zu bestimmende Substanz entweder in einen anderen Wellenbereich verschoben wird oder deren Polarisation geändert wird.

Die Nachteile dieser bekannten Verfahren liegen insbesondere darin, daß die Probe häufig den Test störende Komponenten enthält, was eine Probenvorbehandlung zur Beseitigung dieser Substanzen erforderlich macht. Außerdem ist eine aufwendige Optimierung für jeden Parameter erforderlich, z.B. müssen die Enzyme parameterabhängig modifiziert werden. Bei all diesen Tests bestehen einander entgegengesetzte Anforderungen für eine optimale Differenzierung und optimale Empfindlichkeit, da einerseits die Konzentration des partikelförmigen Reagenzes limitiert sein soll, damit die Konkurrenzreaktion mit der Probe sinnvoll möglich ist, und andererseits das partikelförmige Reagenz hochkonzentriert und hochmarkiert sein soll, um eine genügende Signaländerung pro Zeiteinheit zu erzielen. Die Abstimmung dieser Anforderungen führt zu eingeschränkter Empfindlichkeit und Störanfälligkeit, die oft nur durch spezifische Proben-Vorbehandlung beseitigt werden können.

Zur Lösung dieser Probleme wurde in DE-A 38 22 750 ein homogenes Bestimmungsverfahren vorgeschlagen, bei dem die Probelösung mit 3 Rezeptoren $R_1$, $R_2$ und $R_3$ inkubiert wird, von denen $R_1$ und $R_2$ miteinander bindefähig sind und $R_3$ mit der zu bestimmenden Substanz spezifisch bindefähig ist, wobei Rezeptor $R_1$ ein Konjugat aus einem Partner eines spezifisch bindenden Paares P und einer Substanz S ist, die der zu bestimmenden Substanz entspricht oder ein Derivat davon ist oder mindestens ein Epitop der zu bestimmenden Substanz aufweist, $R_2$ ein Rezeptor ist, der mindestens zwei Bindungsstellen für den spezifisch bindenden Partner aufweist und $R_3$ ein Rezeptor ist, der mindestens zwei Bindungsstellen aufweist, von denen mindestens eine spezifisch mit einem Epitop der zu bestimmenden Substanz bzw. von S bindet. Bei Inkubation der Probelösung mit diesen drei Rezeptoren konkurriert die nachzuweisende Substanz mit dem Rezeptor $R_1$ um Bindung an Rezeptor $R_3$ und Rezeptor $R_2$ bindet mit Rezeptor $R_1$. Nur wenn es zur Bindung der Rezeptoren $R_1$, $R_2$ und $R_3$ kommt, tritt eine Agglutination auf, die photometrisch verfolgt werden kann. Die Bindung der nachzuweisenden Substanz an Rezeptor $R_3$ verhindert die Agglutination, so daß die Agglutination ein indirektes Maß für den Gehalt an nachzuweisender Substanz ist. Dieses Verfahren ist geeignet zum Nachweis von immunologisch aktiven Substanzen wie Antigenen, Antikörpern und Haptenen. Für den Nachweis von Haptenen wird als Rezeptor $R_1$ ein Konjugat eines Partners eines spezifisch bindenden Paares und eines Haptens verwendet. In einer besonders vorteilhaften Ausführungsform wird als Rezeptor $R_1$ ein Konjugat aus Biotin und Substanz S, als Rezeptor $R_2$ mit Streptavidin beschichteter Latex und als Rezeptor $R_3$ ein mit der nachzuweisenden Substanz bindefähiger Antikörper verwendet. An den mit Streptavidin beschichteten Latex bindet das Biotin-Hapten-Konjugat über den Biotin-Anteil. Der Antikörper kann mit dem Hapten-Biotin-Konjugat über den Hapten-Anteil binden. Wenn nun an dem Antikörper zwei Komplexe aus Streptavidin-beschichtetem Latex und Biotin-Hapten-Konjugat binden, so tritt eine Trübung auf, die ausgewertet werden kann. Die Trübung tritt dabei um so langsamer auf, je größer die Analysemenge ist und je geringer die Solvatisierung des Konjugats ist.

Für den Nachweis von Haptenen in Verfahren der beschriebenen Art müssen somit Konjugate aus einem Hapten und Biotin zur Verfügung gestellt werden. Hapten-Biotin-Konjugate sind an sich seit längerem bekannt. So wird beispielsweise in EP-A-315 317 ein sogenanntes zweizähniges Konjugat beschrieben, das aus einem immunologisch aktiven Molekül und einem spezifisch bindenden Partner, die über einen Spacer verbunden sind, besteht. Es wurden nun Untersuchungen angestellt, inwieweit sich die Länge des Spacers auf die Eigenschaften des Konjugats auswirkt. Dabei wurde in dieser Literaturstelle festgestellt, daß die Konjugate eine optimale Wirksamkeit aufweisen, wenn die Spacerlänge mehr als 22,2 Å beträgt, was in etwa einer Kettenlänge von 18 Atomen entspricht, daß aber eine Kettenlänge von mehr als 20 Atomen wiederum die Empfindlichkeit vermindert. Darüber hinaus wird angegeben, daß die Anwesenheit von mehr als 5 Heteroatomen nachteilig ist. Mit diesen Konjugaten wurden jedoch auch noch keine endgültig befriedigenden Ergebnisse erzielt.

In EP 0 310 361 werden dreizähnige Konjugate ("Tridentate") beschrieben, wobei drei funktionelle Gruppen durch einen verzweigten Spacer miteinander verknüpft sind. Als optimaler Abstand zwischen je zwei der funktionellen Gruppen wird beispielsweise für Theophyllin/Biotin eine Spacerlänge entsprechend 20-22 Atomen angegeben. Bifunktionelle Konjugate mit mehr als 22 Atomen Spacerlänge werden nicht offenbart.

Es war nun Aufgabe der Erfindung, bifunktionelle Hapten-Biotin-Konjugate zur Verfügung zu stellen, die für homogene Immunoassay-Verfahren geeignet sind, die eine verbesserte Empfindlichkeit aufweisen und bei denen die Geschwindigkeit der Reaktion erhöht ist.

Diese Aufgabe wird gelöst durch bifunktionelle Hapten-Biotin-Konjugate, welche dadurch gekennzeichnet sind, daß das Hapten mit dem Biotin über einen linearen Spacer verbunden ist, der in der Kette 26 bis 40 Atome aufweist und als Bestandteil der Kette mindestens 5 Heteroatome enthält.

Überraschenderweise wurde festgestellt, daß durch Einsatz der erfindungsgemäß definierten Hapten-Biotin-Konjugate eine deutliche Signalverbesserung gegenüber bekannten Konjugaten erreicht werden kann, durch eine verbesserte Solvatisierung des Spacers das Auftreten von unspezifischen Bindungen vermindert werden kann, die Geschwindigkeit der Reaktion erhöht und die Testperformance verbessert werden kann.

Erfindungsgemäß werden bifunktionelle Hapten-Biotin-Konjugate zur Verfügung gestellt, bei denen das Hapten und das Biotin-Molekül über einen linearen Spacer verbunden sind, der eine Kettenlänge von 26 bis 40 Atomen aufweist und als Bestandteil der Kette mindestens 5 Heteroatome enthält.

Die Heteroatome des Spacers können die in organischen Molekülen vorkommenden Heteroatome wie Stickstoff, Sauerstoff, Schwefel, Phosphor etc. sein. Bevorzugt enthält der Spacer als Heteroatome Stickstoff- und Sauerstoffatome. Die Anzahl der Heteroatome muß mindestens 5 betragen. Vorteilhaft ist ein höherer Anteil an Heteroatomen, wobei der Anteil der Heteroatome so groß sein kann, daß jedes dritte Atom im Spacer ein Heteroatom ist. So kann beispielsweise als Spacer ein Polyethylenoxid der angegebenen Kettenlänge verwendet werden.

Die Spacerlänge liegt im Bereich von 26 bis 40 Atomen, wobei nur die in der Kette vorhandenen Atome gezählt werden. Besonders vorteilhafte Ergebnisse werden mit Spacern erhalten, die mehr als 30 Atome aufweisen.

Die Herstellung der erfindungsgemäßen Konjugate kann entweder erfolgen, indem das Hapten und das Biotin mit einem bifunktionellen Spacermolekül umgesetzt werden, wobei die am Hapten und im Biotinmolekül vorhandenen funktionellen Gruppen mit den funktionellen Gruppen des Spacermoleküls reagieren. Eine andere Möglichkeit besteht darin, das Hapten und/oder das Biotinmolekül zu derivatisieren und anschließend die Derivate gegebenenfalls noch mit einem Spacermolekül umzusetzen. Die Derivate und Spacermoleküle werden dann wiederum so ausgesucht, daß ein Spacer der gewünschten Länge und mit der gewünschten Anzahl an Heteroatomen entsteht.

Die Derivatisierung von Hapten und Biotin erfolgt in an sich bekannter Weise. Als Spacer sind homo- oder hetero-bifunktionelle Linker geeignet, wie Dicarbonsäuren, Diamine, Aminosäuren, Mercaptocarbonsäuren und Halogencarbonsäuren. Bevorzugt werden Spacer verwendet, die aus Sukzinat, Glutarat, Suberat, Ethylendiamin, Propylendiamin, 1,8-Diamino-3,6-dioxaoctan, 1,12-Diamino-4,9-dioxadodecan, Aminobuttersäure, Aminocapronsäure, Thioglykolsäure, Thiopropionsäure, Bromessigsäure und/oder Jodessigsäure aufgebaut sind. Diese Synthesebausteine müssen so zusammengesetzt werden, daß ein Spacer der gewünschten Länge mit der gewünschten Anzahl an Heteroatomen entsteht.

Mit den erfindungsgemäßen Hapten-Biotin-Konjugaten wird eine verbesserte Solvatisierung erzielt, die zu einer verkürzten Reaktionsdauer und damit zu einer Erhöhung der Kapazität führt.

Ein weiterer Gegenstand der Erfindung ist die Verwendung der Hapten-Biotin-Konjugate in einem kompetitiven homogenen Immunoassay, bei dem die bei der Reaktion auftretende Agglutination durch turbidimetrische oder nephelometrische Messungen ausgewertet wird.

Die Erfindung wird durch die nachfolgenden Beispiele erläutert. Die Detektion der Substanzen auf den DC-Platten erfolgt durch Fluoreszenzlöschung bei 254 nm (Kieselgel 60 $F_{254}$, Fa.Merck, Nr.5748 bzw. Kieselgel RP-18, Fa.Merck, Nr.15685) oder bei Biotin-haltigen Verbindungen durch Besprühen mit $H_2SO_4$/4-

Dimethylamino-zimtaldehyd. Mischung A/B 1/1 (v/v):

A) 2% $H_2SO_4$ in Ethanol

B) 0,2% 4-Dimethylamino-zimtaldehyd in Ethanol, wobei anschließend 10 min bei 100°C getrocknet wurde.

**Beispiel 1**

Herstellvorschrift für 5-(1-Methylxanthin-3-yl)pentansäure-N-hydroxysuccinimidester (Theophyllin-3-cb-NHS)

1.a) 1-Methyl-7-(pivaloyloxymethyl)xanthin

83 g 1-Methylxanthin (0,5 mol, Fa.Aldrich, Nr.28,098-4) werden in 3,5 l absolutem DMF gelöst und mit 55 g wasserfreiem Natriumcarbonat versetzt. Anschließend läßt man unter Rühren bei 20°C innerhalb 30 min 80 ml Pivalinsäurechlormethylester (Fa.Aldrich, Nr.14,118-6) in 300 ml absolutem DMF zutropfen. Nach 48 Stunden Reaktionszeit wird über eine Filternutsche abgesaugt und das Filtrat im Hochvakuum eingedampft. Den öligen Rückstand digeriert man mit 500 ml Diisopropylether bis sich das Produkt verfestigt und saugt anschließend ab. Man nimmt den Feststoff in 500 ml Chloroform auf, filtriert von ungelösten Betandteilen ab und dampft die Lösung ein. Der Rückstand wird mit ca 250 ml Petrolether digeriert, das feste Produkt abgesaugt und im Exsikkator getrocknet.

Ausbeute: 24.8 g (18 % d.Th.).

DC: Kieselgel, Essigester; $R_f$ = 0.58.

1.b) 3-Ethyloxycarbonylbutyl-1-methyl-7-(pivaloyloxymethyl)-xanthin

22,4 g (80 mmol) der unter 1a) erhaltenen Verbindung werden in 300 ml absolutem DMF gelöst und mit 17 g (160 mmol) wasserfreiem Natriumcarbonat und 33,6 g ( = 28 ml, 160 mmol) 5-Brompentansäureethylester versetzt. Man läßt 3 Tage bei 20°C rühren, dann verdünnt man mit 3 l Essigester, filtriert die Lösung und wäscht sie dreimal mit je 1 l Wasser. Die Lösung trocknet man mit 100 g wasserfreiem Natriumsulfat und zieht das Lösungsmittel am Rotationsverdampfer ab. Das verbleibende ölige Produkt kann in dieser Form zur nächsten Stufe eingesetzt werden.

Ausbeute: 44 g zähes Öl (enthält größere Mengen an Lösungsmittel).

DC: Kieselgel, Essigester; $R_f$ = 0.75 .

1.c) 3-Carboxybutyl-1-methylxanthin (Theophyllin-3-cb)

Die gesamten 44 g des unter 1b) erhaltenen öligen Produkts werden in 2,8 l 2n NaOH 2 h unter Rückfluß gerührt. Danach läßt man abkühlen und stellt den pH-Wert der Lösung mit konz. HCl auf 5. Das dabei ausfallende Produkt wird abgesaugt, mit Wasser gewaschen und im Exsikkator über Calciumchlorid getrocknet.

Ausbeute: 10.6 g (50 % d.Th.).

DC: Kieselgel, Essigester/Methanol 9/1 (v/v); $R_f$ = 0,37.

1.d) 5-(1-Methylxanthin-3-yl)pentansäure-N-hydroxysuccinimidester (Theophyllin-3-cb-NHS)

5,3 g (20 mmol) der unter 1c) erhaltenen Theophyllin-Carbonsäure werden in 150 ml absolutem DMF gelöst und mit 2,76 g (24 mmol) N-Hydroxysuccinimid und 4,95 g (24 mmol) Dicyclohexylcarbodiimid versetzt. Man läßt 24 Stunden bei 20°C rühren, danach saugt man den ausgefallenen Dicyclohexylharnstoff ab, dampft das Filtrat im Vakuum ein und löst erneut in 100 ml DMF. Nach Filtration wird die Lösung wiederum eingedampft und der Rückstand mit 100 ml Isopropanol digeriert. Man saugt das feste Produkt ab und trocknet im Exsikkator.

Ausbeute: 6,0 g farbloses, feinkristallines Pulver (83 % d.Th.).

DC: Kieselgel RP-18, Nitromethan/Ethanol 9/1 (v/v); $R_f$ = 0,82 .

**Beispiel 2**

Herstellung von Theophyllin-3-cb-NH-NH-Biotin

72,7 mg (0,2 mmol) Theophyllin-3-cb-NHS aus Beispiel 1 werden in 20 ml absolutem DMF gelöst und mit 77,5 mg (0,3 mmol) Biotin-Hydrazid (Fa.Sigma Chemie, Nr. B7639) und 100 μg 4-(N,N-Dimethylamino)-pyridin (Fa.Merck, Nr.820499) versetzt. Man läßt 4 Tage bei 20°C rühren, dann wird das Lösungsmittel im Hochvakuum entfernt und der halbfeste Rückstand mit ca. 5 ml Methanol digeriert. Das feste Produkt wird abgesaugt und an der Hochvakuumpumpe getrocknet.

Ausbeute: 66 mg farbloses Pulver (65 % d.Th.).

DC: Kieselgel, Chloroform/Methanol 2/1 (v/v); $R_f = 0,6$.

**Beispiel 3**

Herstellung von Theophyllin-3-cb-1,8-Diamino-3,6-dioxaoctan-Biotin

182 mg (0,5 mmol) Theophyllin-3-cb-NHS aus Beispiel 1 werden in 15 ml absolutem DMF gelöst und zusammen mit 206 mg (0,55 mmol) Biotin-1,8-diamino-3,6-dioxaoctan (Biotin-DADOO) (Fa.Boehringer Mannheim, Nr.1112 074) 16 h bei 20°C gerührt. Dann entfernt man das Lösungsmittel im Hochvakuum und kristallisiert den Rückstand aus wenig Methanol/Chloroform 1/1 (v/v) um. Das Produkt wird an der Hochvakuumpumpe getrocknet.
Ausbeute: 128 mg farbloses feinkristallines Pulver(41 % d.Th.).
DC: Kieselgel, Chloroform/Methanol 2/1 (v/v); $R_f = 0,57$.

**Beispiel 4**

Theophyllin-3-cb-DADOO-X-Biotin

4.a) Biotin-X-DADOO
7,33 ml (50 mmol) 1,8-Diamino-3,6-dioxaoctan (DADOO, Fa.Merck, Nr.818116) werden in 45 ml Dioxan/0,1 m Kaliumphosphat-Puffer, pH 8,5 2/1 (v/v) gelöst. Unter Rühren bei 20°C läßt man im Verlauf von 30 min eine Lösung von 1,14 g (2,5 mmol) Biotin-e-aminocapronsäure-N-hydroxy-succinimidester (Fa.Boehringer Mannheim, Nr.1003 933) im gleichen Lösungsmittelgemisch zutropfen. Man läßt 5 Stunden weiterrühren, dann wird die Lösung eingedampft und der ölige Rückstand in wenig Methanol gelöst. Unter kräftigem Rühren läßt man in 200 ml Essigester einfließen, wobei sich ein weißlich-gelber Niederschlag bildet. Man saugt ab, löst den Niederschlag in möglichst wenig Methanol/Ammoniak 9/1 (v/v) und reinigt über eine Kieselgel-Säule (5 x 30 cm), Eluent Methanol/Ammoniak 9/1 (v/v). Die entsprechenden Fraktionen werden vereinigt, eingedampft und das feste Produkt an der Hochvakuumpumpe getrocknet.
Ausbeute: 730 mg farbloses Pulver (60 % d.Th.).
DC: Kieselgel, Methanol/Ammoniak 9/1 (v/v); $R_f = 0,46$.
4.b) Theophyllin-3-cb-DADOO-X-Biotin
182 mg (0,5 mmol) Theophyllin-3-cb-NHS aus Beispiel 1 werden in 20 ml absolutem DMF gelöst und mit 245 mg (0,5 mmol) Biotin-X-DADOO und 69 µl (0,5 mmol) Triethylamin versetzt. Man läßt 16 Stunden bei 20°C rühren, dann dampft man das Lösungsmittel im Vakuum ab und reinigt das Produkt durch Chromatographie an einer Kieselgel-Säule (2.8 x 33 cm), Eluent: Chloroform/Methanol 2/1 (v/v). Die entsprechenden Fraktionen werden vereinigt, das Lösungsmittel wird im Vakuum entfernt und der Rückstand mit 20 ml Diisopropylether digeriert. Das feste Produkt wird im Exsikkator über $CaCl_2$ getrocknet.
Ausbeute: 215 mg farbloses Pulver (58 % d.Th.).
DC: Kieselgel, Chloroform/Methanol 2/1 (v/v); $R_f = 0,60$

**Beispiel 5**

Theophyllin-3-cb-DADOD-X-Biotin

5.a) Theophyllin-3-cb-DADOD
4,24 ml (20 mmol) 1,12-Diamino-4,9-dioxadodecan (DADOD, Fa.Aldrich, Nr.22,744-7) werden in 10 ml absolutem DMF gelöst und im Verlauf von 30 min tropfenweise mit einer Lösung von 363 mg (1 mmol) Theophyllin-3-cb-NHS aus Beispiel 1 in 20 ml absolutem DMF versetzt. Nach wenigen Minuten bildet sich ein flockiger Niederschlag. Man läßt 3 Stunden bei 20°C weiterrühren, dann wird filtriert und das Filtrat im Vakuuum eingedampft. Man digeriert mit 50 ml Essigester, dekantiert das Lösungsmittel und löst den Rückstand in wenig DMF. Die Lösung läßt man unter kräftigem Rühren in 50 ml Essigester einfließen. Das hierbei ausfallende Rohprodukt wird abgesaugt und durch Chromatographie an einer Kieselgel-Säule (5 x 35 cm), Eluent: Chloroform/Methanol/Ammoniak 6/3/1 (v/v/v) gereinigt. Die entsprechenden Fraktionen werden vereinigt, das Lösungsmittel im Vakuum entfernt und das verbleibende Produkt aus Wasser lyophilisiert. Man trocknet im Exsikkator über $CaCl_2$.
Ausbeute: 240 mg farbloses Pulver (53 % d.Th.).

DC: Kieselgel, Chloroform/Methanol/Ammoniak 6/3/1 (v/v/v); $R_f$ = 0,42 .

5.b) Theophyllin-3-cb-DADOD-X-Biotin

225 mg (0,5 mmol) Theophyllin-3-cb-DADOD werden in 40 ml absolutem DMF gelöst und mit 227 (0,5 mmol) Biotin-e-aminocapronsäure-N-hydroxysuccinimidester versetzt. Nach Zugabe von 69 $\mu$l (0,5 mmol) Triethylamin läßt man 6 Stunden bei 20°C rühren. Das Lösungsmittel wird im Hochvakuum entfernt, der Rückstand in möglichst wenig Chloroform/Methanol 1/1 (v/v) gelöst und auf eine Kieselgel-Säule (5 x 35 cm) gegeben. Man eluiert mit Chloroform/Methanol 2/1 (v/v). Die entsprechenden Fraktionen werden vereinigt, das Lösungsmittel im Vakuum entfernt und Theophyllin-3-cb-DADOD-X-Biotin aus Wasser lyophilisiert.

Ausbeute: 310 mg farbloses Pulver (78 % d.Th.).

DC: Kieselgel, Chloroform/Methanol 2/1 (v/v); $R_f$ = 0,54.

**Beispiel 6**

Theophyllin-3-cb-DADOO-succ-DADOO-Biotin

6.a) Biotin-DADOO-succ

1,5 g (4 mmol) Biotin-DADOO werden in 30 ml Pyridin gelöst und mit 800 mg (8 mmol) Bernsteinsäureanhydrid versetzt. Nach Zugabe von 0,1 mg 4-(N,N-Dimethylamino)-pyridin läßt man 1 d bei 20°C rühren. Anschließend wird das Lösungsmittel im Hochvakuum entfernt. Der Rückstand wird mit 20 ml Essigester digeriert und abgesaugt. Man wäscht mit ca 20 ml Essigester und trocknet das Produkt im Exsikkator über $CaCl_2$.

Ausbeute: 1,4 g farbloses, feinkristallines Pulver (74 % d.Th.).

DC: Kieselgel RP-18, Isopropanol/Wasser/Eisessig 85/14/1 (v/v/v); $R_f$ = 0,74.

6.b) Biotin-DADOO-succ-DADOO

238 mg (0,5 mmol) Biotin-DADOO-succ löst man in 25 ml absolutem DMF und versetzt mit 57 mg (0,5 mmol) N-Hydroxysuccinimid und 69 $\mu$l (0,5 mmol) 2-(4-Morpholinyl)ethylisocyanid (Fa.Merck, Nr.818649). Man läßt 30 min bei 20°C rühren, dann gibt man 1,5 ml (10 mmol) 1,8-Diamino-3,6-dioxaoctan (DADOO, Fa.Merck, Nr.818116) sowie weitere 57 mg N-Hydroxysuccinimid und 69 $\mu$l 2-(4-Morpholinyl)ethylisocyanid zu. Die Lösung wird 16 Stunden weitergerührt, dann wird das Lösungsmittel im Hochvakuum entfernt und das Produkt durch Chromatographie an einer Kieselgel-Säule (5 x 60 cm), Eluent: Chloroform/Methanol/Ammoniak 6/3/1 (v/v/v) aufgereinigt. Die entsprechenden Fraktionen werden vereinigt, das Lösungsmittel entfernt und der Rückstand mit 10 ml Essigester digeriert. Das feste Produkt saugt man ab und trocknet an der Hochvakuumpumpe.

Ausbeute: 195 mg farbloses Pulver (65 % d.Th.).

DC: Kieselgel, Chloroform/Methanol/Ammoniak 6/3/1 (v/v/v); $R_f$ = 0,54.

6.c) Theophyllin-DADOO-succ-DADOO-Biotin

151 mg (0,25 mmol) Biotin-DADOO-succ-DADOO werden zusammen mit 91 mg (0,25 mmol) Theophyllin-3-cb-NHS aus Beispiel 1 in 10 ml absolutem DMF gelöst und mit 35 $\mu$l (0,25 mmol) Triethylamin versetzt. Man läßt 4 Tage bei 20°C rühren, dann wird das Lösungsmittel im Hochvakuum entfernt und das Produkt durch Säulenchromatographie an einer Kieselgel-Säule (3,5 x 40 cm), Eluent: Chloroform/Methanol 2/1 (v/v) abgetrennt. Die entsprechenden Fraktionen werden vereinigt, das Lösungsmittel im Vakuum entfernt und das Produkt mindestens 6 Stunden an der Hochvakuumpumpe getrocknet.

Ausbeute: 105 mg farbloses, zähflüssiges Öl (49 % d.Th.).

DC: Kieselgel, Chloroform/Methanol 2/1 (v/v); $R_f$ = 0,36.

**Beispiel 7**

Bestimmung von Theophyllin

Reagenzien:

Reagenz 1, bestehend aus 0,1 Gew.-% Streptavidin-Latex (Herstellung nach EP-A 0 349 988)

Reagenz 2, bestehend aus $1,5 \times 10^{-6}$ mol/l monoklonalem Antikörper gegen Theophyllin und $4,5 \times 10^{-7}$ mol/l Konjugat aus Theophyllin und Biotin (hergestellt nach Beispiel 6)

3 $\mu$l Probe werden mit 30 ml Reagenz 2 bei 37°C zusammengegeben und 5 min inkubiert. Anschließend werden 500 $\mu$l Reagenz 1 Streptavidin-Latex zugegeben und die Extinktionsänderung innerhalb 4,2 min bestimmt. Tabelle 1 zeigt die bei Verwendung von wäßrigen Theophyllin-Lösungen als Proben erhaltenen Eichwerte.

**Beispiel 8**

Bestimmung von T4

Reagenzien:

Reagenz 1, bestehend aus 100 mmol/l Barbituratpuffer pH 8,5, 2 Gew.-% Dextransulfat, 0,02 Gew.-% Streptavidin-Latex (hergestellt nach EP-A 0 349 988) 4 x $10^{-8}$ mol/l polyklonale Antikörper (IgG gegen T4).

Reagenz 2, bestehend aus 4 x $10^{-8}$ mol/l Konjugat aus T4 und Biotin, hergestellt analog Beispiel 6, wobei anstelle von theophillin-3cb-NHS $T_4$-(Tert.-butyloxycarbonyl)-NHS (Boehringer Mannheim GmbH) T4 eingesetzt wird.

5 $\mu$l Probe und 235 $\mu$l Reagenz 1 werden bei 37°C zusammengegeben und 5 min inkubiert. Anschließend werden 20 $\mu$l Reagenz 2 zugegeben und die Extinktionsänderung innerhalb 2 min bei 405 nm bestimmt. Bei Verwendung von Serumstandards ergeben sich Eichwerte gemäß Tabelle 2.

## Tabelle 1

| Theophyllin [mol/l] | $\Delta$mE/4,2 min |
|---|---|
| $1,0 \cdot 10^{-5}$ | 461 |
| $2,6 \cdot 10^{-5}$ | 282 |
| $5,0 \cdot 10^{-5}$ | 145 |
| $10^{-4}$ | 40 |
| $2,0 \cdot 10^{-4}$ | 4 |

## Tabelle 2

| $T_4$ [$\mu$g/dl] | $\Delta$mE/2 min |
|---|---|
| 0,6 | 335,12 |
| 4,3 | 201,89 |
| 8,5 | 126,23 |
| 14,6 | 64,74 |
| 26,1 | 23,85 |

**Patentansprüche**

**Patentansprüche für folgende Vertragsstaaten : AT, BE, CH, DE, DK, FR, GB, GR, IT, LI, LU, NL, SE**

1. Bifunktionelle Hapten-Biotin-Konjugate,
   **dadurch gekennzeichnet,**
   daß das Hapten mit dem Biotin über einen linearen Spacer verbunden ist, der in der Kette 26 bis 40 Atome aufweist und als Bestandteil der Kette mindestens 5 Heteroatome enthält.

2. Hapten-Biotin-Konjugate nach Anspruch 1,
   **dadurch gekennzeichnet,**
   daß der Spacer 30 bis 36 Atome enthält.

3. Hapten-Biotin-Konjugate nach Anspruch 1 oder 2,
   **dadurch gekennzeichnet,**
   daß der Spacer als Heteroatome N- oder O-Atome enthält.

4. Hapten-Biotin-Konjugate nach einem der vorhergehenden Ansprüche,
   **dadurch gekennzeichnet,**
   daß das Hapten ein Hormon oder ein Arzneimittel ist.

5. Hapten-Biotin-Konjugate nach einem der vorhergehenden Ansprüche,
   **dadurch gekennzeichnet,**
   daß der Spacer 3-cb-1,8-Diamino-3,6-dioxaoctan oder 3-cb-1,12-Diamino-4,9-dioxadodecan enthält.

6. Verwendung der Hapten-Biotin-Konjugate gemäß einem der Ansprüche 1 bis 5 in einem kompetitiven homogenen Immunoassay, bei dem die bei der Reaktion auftretende Agglutination durch turbidimetrische oder nephelometrische Messungen ausgewertet wird.

**Patentansprüche für folgenden Vertragsstaat : ES**

1. Verfahren zur Herstellung bifunktioneller Hapten-Biotin-Konjugate,
   **dadurch gekennzeichnet,**
   daß man das Hapten mit dem Biotin über einen linearen Spacer verbindet, der in der Kette 26 bis 40 Atome aufweist und als Bestandteil der Kette mindestens 5 Heteroatome enthält.

2. Verfahren nach Anspruch 1,
   **dadurch gekennzeichnet,**
   daß man einen Spacer, der 30 bis 36 Atome enthält, verwendet.

3. Verfahren nach Anspruch 1 oder 2,
   **dadurch gekennzeichnet,**
   daß man einen Spacer, der als Heteroatome N- oder O-Atome enthält, verwendet.

4. Verfahren nach einem der vorhergehenden Ansprüche,
   **dadurch gekennzeichnet,**
   daß man als Hapten ein Hormon oder ein Arzneimittel verwendet.

5. Verfahren nach einem der vorhergehenden Ansprüche,
   **dadurch gekennzeichnet,**
   daß man als Spacer 3-cb-1,8-Diamino-3,6-dioxaoctan oder 3-cb-1,12-Diamino-4,9-dioxadodecan verwendet.

6. Verwendung der Hapten-Biotin-Konjugate, die gemäß einem der Ansprüche 1 bis 5 erhalten werden, in einem kompetitiven homogenen Immunoassay, bei dem die bei der Reaktion auftretende Agglutination durch turbidimetrische oder nephelometrische Messungen ausgewertet wird.

**Claims**
**Claims for the following Contracting States : AT, BE, CH, DE, DK, FR, GB, GR, IT, LI, LU, NL, SE**

1. Bifunctional hapten-biotin conjugates,
   **wherein**
   the hapten is linked to biotin via a linear spacer which has 26 to 40 atoms in the chain and contains at least 5 heteroatoms as a component of the chain.

2. Hapten-biotin conjugates as claimed in claim 1,
   **wherein**
   the spacer contains 30 to 36 atoms.

3. Hapten-biotin conjugates as claimed in claim 1 or 2,
   **wherein**

the spacer contains N or O atoms as heteroatoms.

4. Hapten-biotin conjugates as claimed in one of the previous claims,
   **wherein**
   the hapten is a hormone or a drug.

5. Hapten-biotin conjugates as claimed in one of the previous claims,
   **wherein**
   the spacer is 3-cb-1,8-diamino-3,6-dioxaoctane or 3-cb-1,12-diamino-4,9-dioxadodecane.

6. Use of the hapten-biotin conjugates as claimed in one of the claims 1 to 5 in a competitive homogeneous immunoassay in which the agglutination which occurs in the reaction is evaluated by turbidimetric or nephelometric measurements.

**Claims for the following Contracting State : ES**

1. Process for the production of bifunctional hapten-biotin conjugates,
   **wherein**
   the hapten is linked to biotin via a linear spacer which has 26 to 40 atoms in the chain and contains at least 5 heteroatoms as a component of the chain.

2. Process as claimed in claim 1,
   **wherein**
   a spacer which contains 30 to 36 atoms is used.

3. Process as claimed in claim 1 or 2,
   **wherein**
   a spacer which contains N or O atoms as heteroatoms is used.

4. Process as claimed in one of the previous claims,
   **wherein**
   a hormone or a drug is used as the hapten .

5. Process as claimed in one of the previous claims,
   **wherein**
   3-cb-1,8-diamino-3,6-dioxaoctane or 3-cb-1,12-diamino-4,9-dioxadodecane is used as the spacer.

6. Use of the hapten-biotin conjugates which are obtained as claimed in one of the claims 1 to 5 in a competitive homogeneous immunoassay in which the agglutination which occurs in the reaction is evaluated by turbidimetric or nephelometric measurements.

**Revendications**
**Revendications pour les Etats contractants suivants : AT, BE, CH, DE, DK, FR, GB, GR, IT, LI, LU, NL, SE**

1. Conjugués haptène-biotine bifonctionnels, caractérisés en ce que l'haptène est lié à la biotine par l'intermédiaire d'un espaceur linéaire ayant 26 à 40 atomes dans la chaîne et contenant au moins 5 hétéroatomes comme éléments de la chaîne.

2. Conjugués haptène-biotine selon la revendication 1, caractérisés en ce que l'espaceur contient 30 à 36 atomes.

3. Conjugués haptène-biotine selon la revendication 1 ou 2, caractérisés en ce que l'espaceur contient des atomes d'azote ou d'oxygène comme hétéroatomes.

4. Conjugués haptène-biotine selon l'une des revendications précédentes, caractérisés en ce que l'haptène est une hormone ou un médicament.

**5.** Conjugués haptène-biotine selon l'une des revendications précédentes, caractérisés en ce que l'espaceur contient un 3-cb-1,8-diamino-3,6-dioxaoctane ou un 3-cb-1,12-diamino-4,9-dioxadodécane.

**6.** Utilisation des conjugués haptène-biotine selon l'une des revendications 1 à 5 dans un dosage immunologique homogène compétitif dans lequel on évalue par des mesures turbidimétriques ou néphélométriques l'agglutination qui se produit lors de la réaction.

**Revendications pour l'Etat contractant suivant : ES**

**1.** Procédé de préparation de conjugués haptène-biotine bifonctionnels, caractérisé en ce que l'on relie l'haptène à la biotine par l'intermédiaire d'un espaceur linéaire ayant 26 à 40 atomes dans la chaîne et contenant au moins 5 hétéroatomes comme éléments de la chaîne.

**2.** Procédé selon la revendication 1, caractérisé en ce que l'on utilise un espaceur contenant 30 à 36 atomes.

**3.** Procédé selon la revendication 1 ou 2, caractérisé en ce que l'on utilise un espaceur contenant des atomes d'azote ou d'oxygène comme hétéroatomes.

**4.** Procédé selon l'une des revendications précédentes, caractérisé en ce que l'on utilise comme haptène une hormone ou un médicament.

**5.** Procédé selon l'une des revendications précédentes, caractérisé en ce que l'on utilise comme espaceur un 3-cb-1,8-diamino-3,6-dioxaoctane ou un 3-cb-1,12-diamino-4,9-dioxadodécane.

**6.** Utilisation des conjugués haptène-biotine obtenus selon l'une des revendications 1 à 5 dans un dosage immunologique homogène compétitif dans lequel on évalue par des mesures turbidimétriques ou néphélométriques l'agglutination qui se produit lors de la réaction.